# EUROPÄISCHE PATENTSCHRIFT

(11) **EP 1 103 225 B1**
(45) Veröffentlichungstag und Bekanntmachung des Hinweises auf die Patenterteilung: **15.09.2004**
(21) Anmeldenummer: 00810994.4
(22) Anmeldetag: 26.10.2000
(51) Int. Cl.: A61B 17/58

(54) **Chirurgisches Instrument zum Spannen eines Kabelartigen Spannelements**
Surgical instrument for tensioning a cable-like tension element
Instrument chirurgical de mise en tension d'un élément de tension en forme de câble

(30) Priorität: 25.11.1999 EP 99811087; 22.12.1999 EP 99811194
(43) Veröffentlichungstag der Anmeldung: 30.05.2001
(73) Patentinhaber: Centerpulse Orthopedics Ltd., 6340 Baar (CH)
(72) Erfinder: Steiger, Marco, 9212 Amegg (CH)
(74) Vertreter: Manitz, Finsterwald & Partner Gbr

(56) Entgegenhaltungen:
- EP-A- 0 625 336
- CH-A- 303 821
- FR-A- 2 777 449
- US-A- 5 782 831

## Beschreibung

Die Erfindung betrifft ein chirurgisches Instrument zum Spannen eines kabel-, band- oder schnurartigen Spannelements.

Das Spannelement kann beispielsweise ein aus Metalldrähten gebildetes Kabel, ein aus Kunststoff-Filamenten gebildeter Fadenverbund oder ein lineares Flechtwerk von Garnen sein. Ein solches Spannelement wird auch kurz als Band bezeichnet.

Zur chirurgischen Behandlung von Verkrümmungen des Rückgrats (Skoliose) hat Dwyer eine Operationstechnik entwickelt, bei der im Körper des Patienten mittels Kabel und Schrauben eine Folge von benachbarten Wirbeln zu einem versteiften Teil der Wirbelsäule zusammengespannt werden. Bei dieser Operation wird ein Kabelspanngerät verwendet. Aus der GB-A- 1 551 707 ist ein derartiges Gerät bekannt. Dieses Kabelspanngerät umfasst einen ersten hebelartigen Teil und einen zweiten Teil, der als Greiferelement für das Kabel ausgebildet ist. Diese beiden Teile sind über ein Hauptgelenk miteinander verbunden. Der zweite Teil ist ein Hebelsystem, in dem Hebel über drei Gelenke miteinander verbunden sind. Zwei dieser Hebel bilden einen Klemmspalt des Greiferelements.

In der CH-A-303 821 ist ein Spanninstrument für Cerclagedrähte gezeigt. Die beiden Drahtenden greifen annähernd senkrecht zur Längsachse eines Anzeigearms an, der an einem Zughebel gelagert ist und über eine Rückholfeder eine notwendige Gegenkraft erfährt.

In der FR-A-2777449 wird ein alternatives chirurgisches Instrument mit den Merkmalen des Oberbegriffs des Anspruchs 1 zum Spannen von Bändern gezeigt, das verglichen mit dem oben genannten Kabelspanngerät aus weniger gegeneinander beweglichen Einzelteilen zusammengesetzt ist und das als manuell betätigbares Hebelsystem mit folgenden Merkmalen definierbar ist:

Das Hebelsystem besteht aus zwei durch einen dritten Hebel gelenkig verbundenen Betätigungshebeln und zwischen den Hebeln angeordneten Federn. Dabei ist eine stabile Grundstellung des Systems durch die unter mechanischer Spannung stehenden Federn sowie durch zwischen den Betätigungshebeln und dem dritten Hebel wirkenden Bewegungsbegrenzungen gegeben. An einer Spitze des ersten Betätigungshebels ist eine Einlegeöffnung für das Spannelement und an einer Spitze des zweiten Betätigungshebels eine erste Backe zu einem Klemmspalt für das Spannelement angeordnet. Eine zweite Backe zu diesem Klemmspalt befindet sich am dritten Hebel. In der Grundstellung des Systems lässt sich das Spannelement in die Einlegeöffnung des ersten Betätigungshebels sowie in den Klemmspalt am zweiten Betätigungshebel einschieben. Durch ein manuelles Zusammenpressen der Betätigungshebel macht der zweite Betätigungshebel eine Schwenkbewegung gegenüber dem dritten Hebel, aufgrund welcher sich der Klemmspalt verengt, so dass das Spannelement festgeklemmt wird. Durch ein weiteres Zusammenpressen vergrössert sich der Abstand zwischen den Spitzen der beiden Betätigungshebel. Der dritte Hebel ist symmetrisch als Bügel mit zwei Seitenplatten ausgebildet, wobei die Betätigungshebel zwischen den Seitenplatten an Lagerbolzen verschwenkbar angeordnet sind und die Lagerbolzen in Bohrungen der beiden Seitenplatten befestigt sind. An der Scheitelstelle des Bügels befindet sich die Klemmbacke des dritten Hebels. Zusammen mit der anderen Klemmbacke und Teilen der Seitenplatten ist ein seitlich geschlossener Kanal für das Band gegeben. Bei diesem aus drei Hebeln bestehenden System erwies sich die Herstellung der Gelenklager als aufwendig. Außerdem ist die Ermittlung der Bandspannung ungenau, da man von der Elastizität des Bandes und einem bestimmten Spannweg ausgeht, ohne die Abweichungen in der Länge des Bandes und in einer spannungslosen Streckung zu berücksichtigen.

Es ist Aufgabe der Erfindung, ein verbessertes Bandspanngerät zu schaffen. Diese Aufgabe wird durch die Merkmale von Anspruch 1 gelöst.

Das chirurgische Instrument zum Spannen eines kabel-, band- oder schnurartigen Spannelements umfasst ein manuell betätigbares Hebelsystem. Es sind zwei Hebel - der erste und der zweite Betätigungshebel - durch einen dritten Hebel gelenkig verbunden. In einer Grundstellung des Systems ist das Spannelement in eine Einlegeöffnung des ersten und in einen Klemmspalt am zweiten sowie dritten Hebel einlegbar. Einer der Betätigungshebel ist in einem Abschnitt als biegeelastischer Balken ausgebildet. Das Ausmass einer Formänderung des biegeelastischen Balkens ist mit angebauten Messmitteln messbar. Eine Beziehung zwischen dieser Formänderung und einer Zugkraft im Spannelement ist bekannt. Aufgrund dieser Kenntnis und einer Messung der Formänderung ist die Zugkraft einstellbar. Die Grundstellung ist vorzugsweise eine stabile Lage, nämlich aufgrund von mindestens einer zwischen den Hebeln angeordneten Feder oder aufgrund von einem anderen, entsprechende Kräfte ausübenden Mittel.

Der dritte Hebel ist asymmetrisch ausgebildet und weist nur eine Seitenplatte auf. Lagerbolzen für zwei Hebelgelenke sind Teile des dritten Hebels. In einer Ausführungsform ist eine einzige Feder am dritten Hebel befestigt. Eine Klemmbacke des dritten Hebels ist die Grundfläche eines U-förmigen Kanals. Die Feder weist zwei Schenkel auf, die jeweils unter einer mechanischen Spannung an Innenflanken der Betätigungshebel anliegen. Eine Klemmbacke des zweiten Hebels taucht in den U-förmigen Kanal ein. Eine zwischen dem zweiten und dem dritten Hebel wirkende Bewegungsbegrenzung ist durch einen separat einfügbaren Anschlagstift gegeben.

Die abhängigen Ansprüche 2 bis 11 betreffen vorteilhafte Ausführungsformen des erfindungsgemässen Instruments.

Nachfolgend wird die Erfindung anhand der Zeichnungen erläutert. Es zeigen:
- Fig. 1: ein erfindungsgemässes Instrument,
- Fig. 2: den vorderen Teil dieses Instruments mit einem unter eine Zugkraft gesetzten Band,
- Fig. 3: ein dritter Hebel des erfindungsgemässen Instruments,
- Fig. 4: den vorderen Teil des Instruments mit festgeklemmtem Band,
- Fig. 5 -6: Ausschnitte jeweils aus einem zweiten Betätigungshebel mit Mitteln zur Durchführung einer Kraftmessung,
- Fig. 7: eine Alternative zum mechanischen Ausführungsbeispiel der Figuren 1 bis 5 und
- Fig. 8: eine weitere Alternative, bei der die Feder durch Magnetelemente substituiert ist.

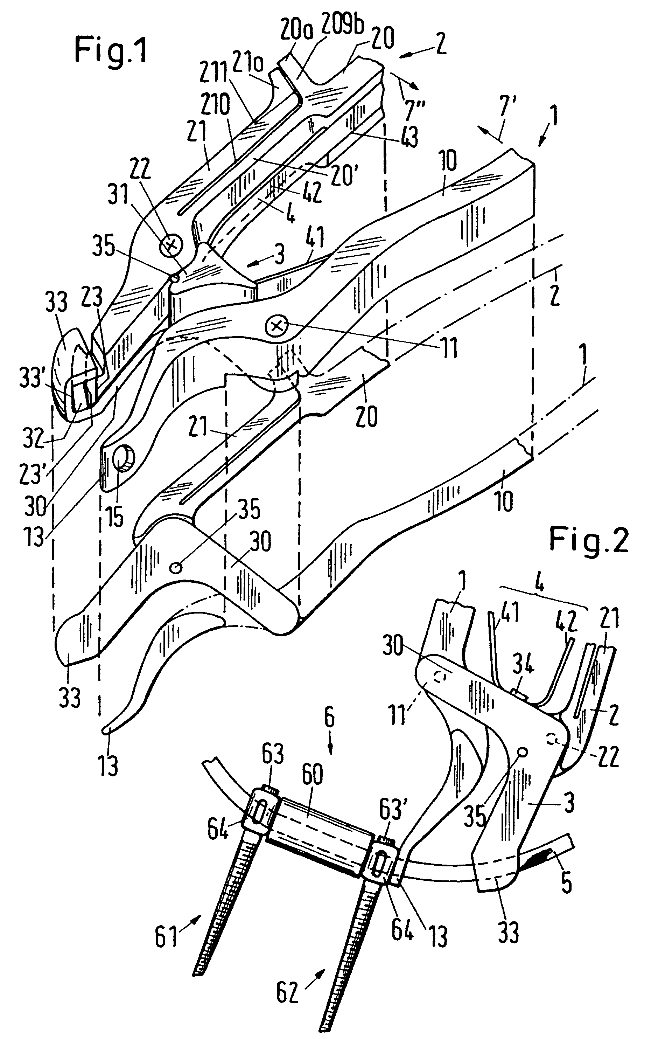

Das in Fig. 1 gezeigte erfindungsgemässe Instrument, das Bandspanngerät, umfasst ein Hebelsystem, das aus zwei Hebeln 1 und 2 besteht, die durch einen dritten Hebel 3 an Gelenken 11 und 22 verbunden sind. Die beiden ersten Hebel 1, 2, nämlich der erste und zweite Betätigungshebel, haben Handgriffe 10 und 20, die in Fig. 1 nur teilweise dargestellt sind. Das Instrument ist asymmetrisch ausgebildet; die sichtbare Seite unterscheidet von der nicht sichtbaren, die in der unteren Hälfte der Fig. 1 als Spiegelbild abgebildet ist (wobei nur die Flächen der untersten Seitenebene dargestellt sind und die Handgriffe 10 und 20 teilweise strichpunktiert angedeutet sind).

Durch eine zwischen den Hebeln 1, 2 und 3 angeordnete Feder 4, die unter einer mechanischen Spannung steht, und Bewegungsbegrenzungen ist eine stabile Grundstellung des Systems gegeben. Eine Bewegungsbegrenzung zwischen dem ersten Betätigungshebel 1 und dem dritten Hebel 3 stellt ein Teil 31 des dritten Hebels 3 her. Eine Bewegungsbegrenzung zwischen dem zweiten Betätigungshebel 2 und dem dritten Hebel 3 stellt ein Anschlagstift 35 her. In Fig. 1 ist eine fiktive Hebelstellung zu sehen, die den ersten Betätigungshebel 1 in einer ausgelenkten Lage und die anderen zwei Hebel 2 und 3 in den Lagen, die sie in der Grundstellung haben, darstellt.

An einer Spitze 13 des ersten Betätigungshebels 1 ist eine Einlegeöffnung 15 für ein kabel-, band- oder schnurartiges Spannelement oder kurz Band 5-siehe Fig. 2 - angeordnet. An einer Spitze 33 des dritten Hebels 3 ist eine erste Backe 33' zu einem Klemmspalt 32 für das Band 5 angeordnet. Eine zweite Backe 23' zu diesem Klemmspalt 32 befindet sich an einem Ende 23 des zweiten Betätigungshebels 2. In der Grundstellung des Systems lässt sich das Band 5 in die Einlegeöffnung 15 des ersten Betätigungshebels 1 sowie in den Klemmspalt 32 am zweiten Betätigungshebel 2 einschieben. Durch ein manuelles Zusammenpressen der Betätigungshebel 1 und 2-durch Pfeile 7' und 7" angedeutet - macht der zweite Betätigungshebel 2 eine kleine Schwenkbewegung gegenüber dem dritten Hebel 3, aufgrund welcher sich der Klemmspalt 32 verengt, so dass das Band 5 festgeklemmt wird. Durch ein weiteres Zusammenpressen ergibt sich eine Spreizbewegung, durch die sich der Abstand zwischen den Spitzen 13 und 33 der beiden Betätigungshebel 1 und 2 vergrössert.

Fig. 2 zeigt den vorderen Teil des erfindungsgemässen Instruments, mit dem das Band 5 durch die genannte Spreizbewegung unter eine Zugkraft gesetzt worden ist. Dabei ist das Band 5 mit einer zylindrischen Schraube 63 im Kopf 64 einer ersten Pedikularschraube 61 fixiert. Das Band 5, das von der ersten Pedikularschraube 61 durch ein Kunststoffrohr 60 (sogenanntes Kissen) zum Kopf 64' einer zweiten Pedikularschraube 62 führt, kann dort im gespannten Zustand mit einer weiteren zylindrischen Schraube 63' fixiert werden. Die aus den Pedikularschrauben 61, 62 und dem Rohr 60 gebildete Einheit 6 wird bei einer Wirbelsäulen-Operation dazu verwendet, zwei benachbarte Wirbel in einem durch die Länge des Rohrs 60 gegebenen Abstand zu fixieren.

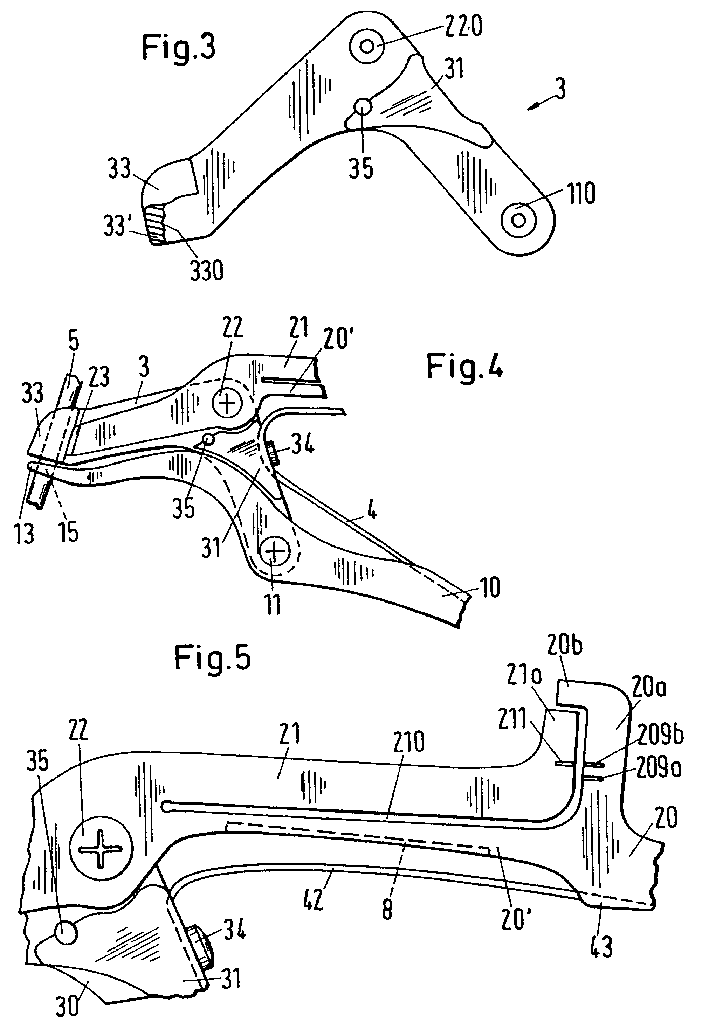

Der dritte Hebel 3 - siehe Fig. 3 - ist asymmetrisch mit nur einer Seitenplatte 30 ausgebildet. Lagerbolzen 110, 220 für die zwei Hebelgelenke 11, 22 sind Teile des dritten Hebels 3. Die Betätigungshebel 1 und 2, die den Bolzen entsprechende Bohrungen aufweisen, werden an den Bolzen 110, 220 angebracht, wobei mit Schrauben drehbare Verbindungen hergestellt werden.

Die Klemmbacke 33' des dritten Hebels 3 bildet die Grundfläche 330 eines U-förmigen Kanals. Die Klemmbacke 23' des zweiten Hebels 2 taucht in den U-förmigen Kanal ein. Die zwischen dem zweiten Betätigungshebel 2 und dem dritten Hebel 3 wirkende Bewegungsbegrenzung ist durch einen separat einfügbaren Anschlagstift 35 gegeben. Der Betätigungshebel 2 kann aus geometrischen Gründen nur mit dem dritten Hebel 3 zusammengesetzt werden, wenn der Anschlagstift 35 noch fehlt.

Die Feder 4 ist am dritten Hebel 3 mit einer Schraube 34 - siehe Fig. 2-befestigt. Sie weist zwei Schenkel 41 und 42 auf, die jeweils unter einer mechanischen Spannung an Innenflanken 43 der Betätigungshebel 1 und 2 anliegen, wobei die Schenkel 41 und 42 in Nuten der Innenflanken 43 gleiten können. Die Einlegeöffnung 13 des ersten Betätigungshebels 1 ist eine Öse; sie kann aber auch gabelartig als Lücke zwischen zwei Zinken ausgebildet sein. Die Backen 23' und 33' des Klemmspalts 32 weisen mit Vorteil auf Klemmflächen 230, 330 Rippungen auf, wobei Rippungskanten vorzugsweise quer zur Richtung der auf das Band 5 wirkenden Zugkraft orientiert sind.

Fig. 4 zeigt den vorderen Teil des Instruments mit festgeklemmtem Band 5. Der Betätigungshebel 1 ist noch in der Grundstellung. Der Anschlagstift 35 weist einen Abstand zum Betätigungshebel 2 auf.

Der Betätigungshebel 2 ist auf der Seite des Handgriffs 20 längs eines Abschnitts als biegeelastischer Balken 20' ausgebildet: siehe Figuren 1 und 5. Das Ausmass einer Formänderung des Balkens 20' ist messbar. Eine Beziehung zwischen dieser Formänderung und einer Zugkraft im Band 5 ist bestimmbar und als bekannt vorausgesetzt. Bei der Betätigung des Bandspanngeräts ist aufgrund dieser Kenntnis und einer Messung der Formänderung die Zugkraft im Band 5 kontrollierbar und somit einstellbar. Der biegeelastische Balken 20' ist neben einem Referenzbalken 21 angeordnet. Die beiden Balken 20' und 21 erstrecken sich parallel zueinander längs eines Spalts 210. Quer zum Spalt 210 sind Segmente 20a und 21a mit den Balken 20' und 21 verbunden. Es sind Markierungen 209a, 209b, 210 auf den Segmenten 20a bzw. 21 a zur Bestimmung der Formänderung angebracht. Die Markierungslinien 209b und 210 werden beispielsweise so angebracht, dass diese bei Erreichen einer Zugkraft im Band 5, die nicht überschritten werden soll, auf eine gemeinsame Gerade - vgl. Fig. 5 - zu liegen kommen. Die Markierungslinie 209a kann einen Nullpunkt angeben, für den das Band 5 bereits im Klemmspalt 32 fixiert ist, ohne dass dabei schon eine Zugkraft wirkt. Es kann vorgesehen werden, die Markierungen auf separat montierbaren Blättchen anzubringen. Diese Blättchen können einem Nullpunkt entsprechend, der empirisch festgestellt wird, montiert werden.

Das Segment 20a am biegeelastischen Balken 20' kann geometrisch so ausgebildet sein, dass sich eine Biegebeschränkung unter einem Zusammenwirken mit dem Segment 21a am Referenzbalken 21 ergibt. Ein Beispiel einer solchen Biegebeschränkung ist in Fig. 5 illustriert: das Ende 20b des Segments 20a ist hakenförmig ausgebildet.

Auf dem biegeelastischen Balken 20' kann ein Sensor, insbesondere ein Dehnmessstreifen 8 angebracht sein. Der Sensor ist Teil eines elektrischen und/oder elektronischen Messgeräts zum Anzeigen der im Band 5 wirkenden Zugkraft (nicht dargestellt). Das Messgerät kann am oder im Betätigungshebel, auf dem der Sensor angeordnet ist, integriert sein.

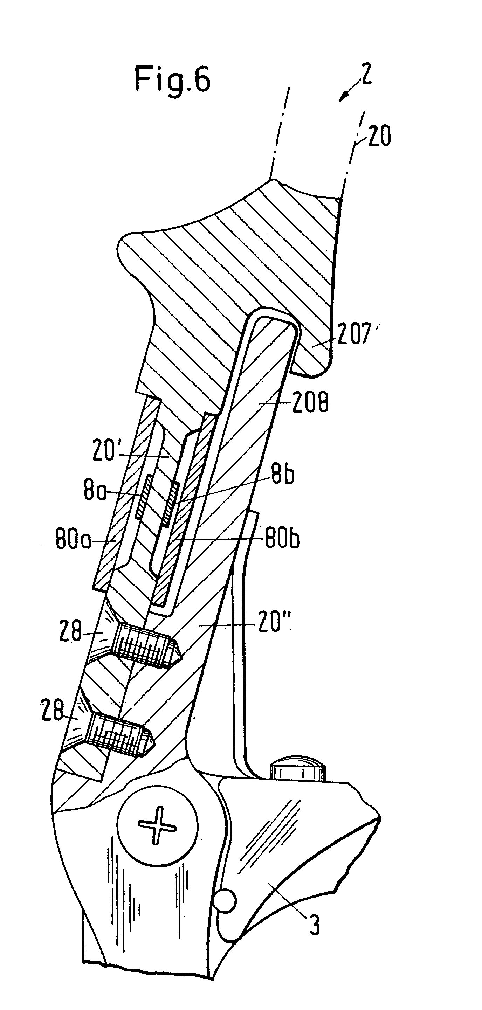

Die Fig. 6 zeigt ein zweites Beispiel mit Dehnmessstreifen. Der Betätigungshebel 2 ist aus einem Handgriffteil 20 und einem Vorderteil 20" mit zwei Schrauben 28 zusammengesetzt. An zwei Flanken eines biegeelastischen Balkens 20' sind zwei Dehnmessstreifen 8a, 8b angebracht. Zur Kapselung dieser Sensoren dienen zwei weiche Abdeckschichten 80a, 80b, deren Einfluss auf die Formänderung des Balkens 20' praktisch vernachlässigbar ist. Ein Nocken 207 und ein Balken 208 wirken als Biegebeschränkung zwecks eines Überlastschutzes.

Zu Messketten zwischen den Sensoren und Anzeigegeräten können noch folgende Bemerkungen anfügt werden: Im Anzeigegerät erfolgt eine Signalauswertung. Die Anzeige des Ergebnisses dieser Signalauswertung erfolgt optisch und/oder akustisch. Ist der Sensor ein Dehnmessstreifen, so ist eine Stromversorgung erforderlich. Ein Messwertverstärker für ein Spannungsoder Stromsignal (Dehnmessstreifen) kann im Instrument oder im Anzeigegerät integriert sein. Die Dehnmessstreifen können als Halb- oder Vollbrücke geschaltet sein. Ein Kabel für die Verbindung zwischen Messwertverstärker und Anzeigegerät muss wasserdampfsterilisierbar sein und einen Knickschutz aufweisen. Bevorzugte Werkstoffe für das Isolationsmaterial sind Silikongummi und/oder Teflon. Schnittstellen Instrument-Kabel und Kabel-Anzeigegerät müssen für medizinaltechnische Anwendungen geeignet sein, d. h. in Form von Steckverbindungen vorliegen, die sterilisierbar und hermetisch abgedichtet sind. Die Schnittstelle Instrument-Kabel kann statt lösbar auch fest sein.

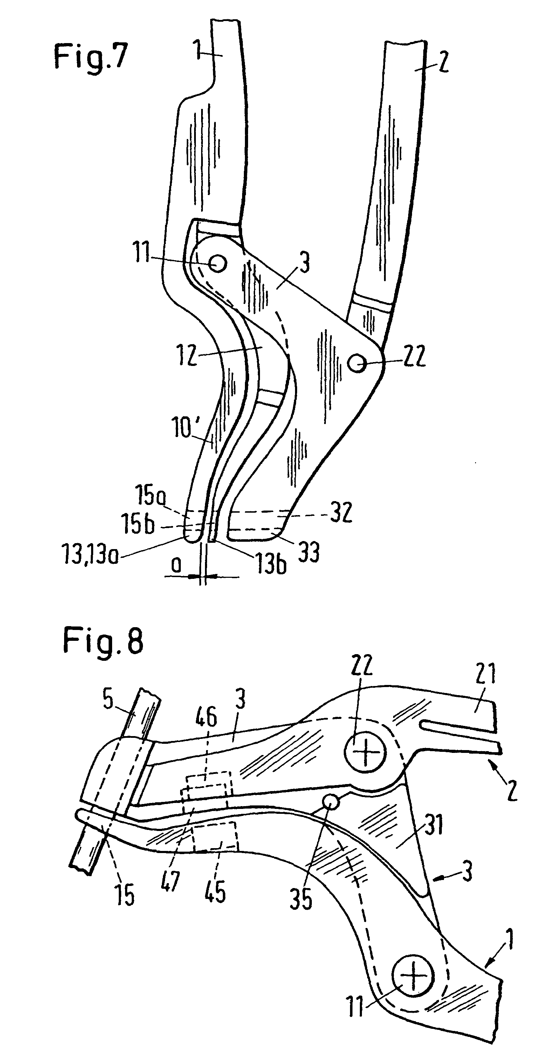

Eine Alternative zum mechanischen Ausführungsbeispiel der Figuren 1 bis 5, d. h. dem Beispiel des erfindungsgemässen Bandspanngeräts, bei dem mit einer mechanischen Anzeige die Zugkraft im zu spannenden Band 5 kontrolliert wird, ist in Fig. 7 gezeigt. Bei diesem Beispiel ist ein als biegeelastischer Balken 10' ausgebildeter Abschnitt Teil des ersten Betätigungshebels 1 und zwar ein Teil im sigmoid geformtem Vorderbereich zwischen der Spitze 13 und dem Gelenk 11. Ein Referenzbalken 12 ist ein zweiter Teil des Vorderbereichs. An der Basis dieses Referenzbalkens 12 befindet sich das Gelenk 11, das die Verbindung zwischen dem Betätigungshebel 1 und dem dritten Hebel 3 herstellt. Beim Spannen eines nicht dargestellten Bands 5 (vgl. Fig. 2) verkleinert sich ein Abstand a zwischen den Balken 10' und 12 an deren Spitzen 13a bzw. 13b (proportional zur Zugkraft). Ein in der Grundstellung gegebener Abstand a wird mit Vorteil so gross festgelegt, dass sich die Balken 10' und 12 beim Erreichen einer gewünschten Zugkraft berühren, d. h. der Abstand a Null wird. Beide Balken 10' und 12 enthalten an ihren Spitzen 13a, 13b Einlegeöffnungen 15a bzw. 15b für das Band 5. Da bei einer Operation der Chirurg den Abstand a nur schwer erkennen kann, ist das erste mechanische Ausführungsbeispiel der in Fig. 7 gezeigten Alternative vorzuziehen.

In Fig. 8 ist eine weitere Alternative dargestellt, bei der die Feder 4 durch Magnetelemente 45, 46 und 47 substituiert ist, die mit magnetischen Anziehungskräften aufeinander einwirken. Mindestens eines dieser Magnetelemente ist ein Permanentmagnet 45; das andere oder die anderen Magnetelemente 46 und 47 sind ferromagnetisch. Der Permanentmagnet 45 oder eines der Permanentmagnete ist im ersten Betätigungshebel 1, dort im Bereich zwischen der Einlegeöffnung 15 und dem Hebelgelenk 11, angeordnet; und das andere oder die anderen Magnetelemente 46 und 47 sind an entsprechenden Stellen im zweiten Betätigungshebel bzw. im dritten Hebel angeordnet.

## Patentansprüche

1. Chirurgisches Instrument zum Spannen eines kabel-, band- oder schnurartigen Spannelements (5), ein manuell betätigbares Hebelsystem umfassend, mit zwei durch einen dritten Hebel (3) gelenkig verbundenen Hebeln (1, 2) - dem ersten und dem zweiten Betätigungshebel -, wobei in einer Grundstellung des Systems das Spannelement in eine Einlegeöffnung (15) des ersten und in einen Klemmspalt (32) am zweiten sowie dritten Hebel einlegbar ist,
**dadurch gekennzeichnet, dass** einer der Betätigungshebel (2) in einem Abschnitt als biegeelastischer Balken (20', 10') ausgebildet ist, am biegeelastischen Balken Mittel zum Messen der Formänderung des Balkens vorgesehen sind, eine Beziehung zwischen dieser Formänderung und einer Zugkraft im Spannelement bekannt ist und aufgrund dieser Kenntnis und einer Messung (209, 211; 8) der Formänderung die Zugkraft einstellbar ist.

2. Instrument nach Anspruch 1, **dadurch gekennzeichnet, dass** die Grundstellung aufgrund von mindestens einer zwischen den Hebeln angeordneten Feder (4) oder aufgrund von einem anderen, entsprechende Kräfte ausübenden Mittel (45, 46, 47) eine stabile Lage darstellt.

3. Instrument nach Anspruch 2, **dadurch gekennzeichnet, dass** der dritte Hebel (3) asymmetrisch, nur eine Seitenplatte (30) aufweisend, ausgebildet ist, Lagerbolzen (110, 220) für zwei Hebelgelenke (11, 22) Teile des dritten Hebels sind, eine einzige Feder (4) am dritten Hebel befestigt ist und eine Klemmbacke (33') des dritten Hebels die Grundfläche eines U-förmigen Kanals (32, 33) ist, wobei die Feder zwei Schenkel (41, 42) aufweist, die jeweils unter einer mechanischen Spannung an Innenflanken (43) der Betätigungshebel (1, 2) anliegen, eine Klemmbacke (23') des zweiten Hebels (2) in den U-förmigen Kanal eintaucht und eine zwischen dem zweiten und dem dritten Hebel wirkende Bewegungsbegrenzung durch einen separat einfügbaren Anschlagstift (35) gegeben ist.

4. Instrument nach Anspruch 3, **dadurch gekennzeichnet, dass** die Feder durch Magnetelemente (45, 46, 47) substituiert ist, die mit magnetischen Anziehungskräften aufeinander einwirken, wobei mindestens eines dieser Magnetelemente ein Permanentmagnet (45) ist und das andere oder die anderen Magnetelemente ferromagnetisch sind, der Permanentmagnet oder eines der Permanentmagnete im ersten Betätigungshebel (1), dort im Bereich zwischen der Einlegeöffnung (15) und dem Hebelgelenk (11), angeordnet ist und das andere oder die anderen Magnetelemente an entsprechenden Stellen im zweiten Betätigungshebel (2) bzw. im dritten Hebel (3) angeordnet sind.

5. Instrument nach einem der Ansprüche 1 bis 4, **dadurch gekennzeichnet, dass** der biegeelastische Balken (20') neben einem Referenzbalken (21) angeordnet ist, dass die beiden Balken sich im wesentlichen parallel in einer Richtung erstrecken und dass quer zu der Balkenrichtung Segmente (20a, 21a) mit den Balken verbunden sind, auf denen Markierungen (209a, 209b, 211) zur Bestimmung der Formänderung angebracht sind.

6. Instrument nach Anspruch 5, **dadurch gekennzeichnet, dass** das Segment (20a) am biegeelastischen Balken (20') geometrisch so ausgebildet ist, dass sich eine Biegebeschränkung unter einem Zusammenwirken mit dem Segment (20b) am Referenzbalken (21) ergibt.

7. Instrument nach einem der Ansprüche 1 bis 7, **dadurch gekennzeichnet, dass** für die Mittel zum Messen der Formänderung des Balkens ein Sensor (8), insbesondere ein Sensor mit Dehnmessstreifen (8; 8a, 8b) vorgesehen ist, und dass der Sensor Teil eines elektrischen und/oder elektronischen Messgeräts zum Anzeigen der im Spannelement wirkenden Zugkraft ist.

8. Instrument nach Anspruch 7, **dadurch gekennzeichnet, dass** am Betätigungshebel (2), auf dem der Sensor (8) angeordnet ist, das Messgerät integriert ist.

9. Instrument nach einem der Ansprüche 1 bis 8, **dadurch gekennzeichnet, dass** die Einlegeöffnung (15) des ersten Betätigungshebels (1) eine Öse oder eine Lücke zwischen zwei Gabelzinken ist.

10. Instrument nach einem der Ansprüche 1 bis 9, **dadurch gekennzeichnet, dass** die Backen (23', 33') des Klemmspalts (32) Rippungen auf den Klemmflächen (330) aufweisen, mit einer Orientierung von Rippungskanten, die vorzugsweise quer zur Richtung der Zugkraft verläuft.

11. Instrument nach einem der Ansprüche 1 bis 7, **dadurch gekennzeichnet, dass** für die Mittel (20a, 21a; 8) zum Messen der Formänderung des Balkens eine Anzeige eines Nullpunkts (209a) vorgesehen ist, wobei diese Nullpunkts-Anzeige einstellbar ist.

## Claims

1. Surgical instrument for tensioning a cable-like, a band-like or a cord-like tensioning element (5), comprising a manually actuatable lever system with two levers (1, 2) - the first and the second actuation lever - which are hingedly connected by a third lever (3), with it being possible, in a basic position of the system, to insert the tensioning element into an insertion opening (15) of the first lever and into a clamping gap (32) at the second and third levers,
**characterized in that** one of the actuation levers (2) is formed in a section as a beam (20', 10') which is resilient in bending, and **in that** means are provided at the beam resilient in bending for the measurement of a shape change of the beam, with a relationship between this shape change and a tension in the tensioning element being known and it being possible to set the tension as a result of this knowledge and of a measurement (209, 211; 8) of the shape change.

2. Instrument in accordance with claim 1, **characterized in that** the basic position represents a stable position on the basis of at least one spring (4) arranged between the levers or on the basis of another means (45, 46, 47) exerting corresponding forces.

3. Instrument in accordance with claim 2, **characterized in that** the third lever (3) is designed asymmetrically having only one side plate (30), bearing bolts (110, 220) for two lever hinges (11, 22) are parts of the third lever, a single spring (4) is secured to the third lever and a clamping jaw (33') of the third lever is the base surface of a U-shaped passage (32, 33), with the spring having two limbs (41, 42) which each lie in contact at inner flanks (43) of the actuation levers (1,2) under a mechanical stress, with a clamping jaw (23') of the second lever (2) entering into the U-shaped passage and with a limitation on movement acting between the second and the third lever being realized by a separately insertable abutment pin (35).

4. Instrument in accordance with claim 3, **characterized in that** the spring is substituted by magnetic elements (45, 46, 47) which act on one another with magnetic forces of attraction, with at least one of these magnetic elements being a permanent magnet (45) and the other magnetic element or elements being ferromagnetic, the permanent magnet or one of the permanent magnets being arranged in the first actuating lever (1), in the region between the insertion opening (15) and the lever hinge (11) there, and the other magnetic element or elements being arranged at corresponding positions in the second actuating lever (2) or in the third lever (3).

5. Instrument in accordance claim with any one of claims 1 to 4, **characterized in that** the beam (20') which is resilient in bending is arranged adjacent to a reference beam (21); **in that** the two beams extend substantially parallel in the same direction; and **in that** segments (20a, 21a) are connected to the beams transverse to the beam direction on which markings (209a, 209b, 211) for the determination of the shape change are provided.

6. Instrument in accordance with claim 5, **characterized in that** the segment (20a) at the beam (20') which is resilient in bending is formed geometrically in such a manner that a bending restriction results on cooperation with the segment (20b) at the reference beam (21).

7. Instrument in accordance with any one of claims 1 to 7, **characterized in that** a sensor (8), in particular a sensor with strain gauges (8; 8a, 8b) is provided for the means for measuring the shape change of the beam; and **in that** the sensor is part of an electrical and/or electronic measurement device for indicating the tension which acts in the tensioning element.

8. Instrument in accordance with claim 7, **characterized in that** the measurement device is integrated at the actuation lever (2) on which the sensor (8) is arranged.

9. Instrument in accordance with any one of claims 1 to 8, **characterized in that** the insertion opening (15) of the first actuation lever (1) is an eyelet or a gap between two fork prongs.

10. Instrument in accordance with any one of claims 1 to 9, **characterized in that** the jaws (23', 33') of the clamping gap (32) have ribs on the clamping surfaces (330) with an orientation of rib edges which preferably extends transversely to the direction of the tension.

11. Instrument in accordance with any one of the claims 1 to 7, **characterized in that** a display of a zero-point (209a) is provided for the means (20a, 21a; 8) for measuring the shape change of the beam, with this zero-point display being settable.

## Revendications

1. Instrument chirurgical destiné à tendre un élément de tension (5) du type câble, bande ou cordon, comprenant un système de leviers propre à un actionnement manuel, qui comporte deux leviers (1, 2), à savoir le premier levier d'actionnement et le deuxième levier d'actionnement, qui sont reliés de manière articulée par un troisième levier (3), et dans lequel, dans une position initiale du système, l'élément de tension peut être inséré dans une ouverture d'insertion (15) du premier levier et dans une fente de serrage (32) au niveau du deuxième levier ainsi que du troisième levier,
**caractérisé en ce que** l'un des leviers d'actionnement (2) est réalisé, dans une partie, en tant que barre (20', 10') présentant une élasticité à la flexion, et **en ce que**, au niveau de la barre présentant une élasticité à la flexion, des moyens pour mesurer la déformation de la barre sont prévus, un rapport entre cette déformation et une force de traction dans l'élément de tension étant connu et sur la base de cette connaissance et d'une mesure (209, 211 ; 8) de la déformation, la force de traction peut être réglée.

2. Instrument selon la revendication 1, **caractérisé en ce que** la position initiale constitue une position stable, du fait d'au moins un ressort (4) disposé entre les leviers ou du fait d'un autre moyen (45, 46, 47) exerçant des forces correspondantes.

3. Instrument selon la revendication 2, **caractérisé en ce que** le troisième levier (3) est réalisé de manière asymétrique, présentant uniquement une plaque latérale (30), des tourillons (110, 220) destinés à deux articulations de leviers (11, 22) faisant partie du troisième levier, un seul ressort (4) étant fixé au troisième levier et un mors de serrage (33') du troisième levier constituant la surface de base d'un canal en forme de U (32, 33), et dans lequel le ressort comporte deux branches (41, 42), chacune portant contre les flancs intérieurs (43) des leviers d'actionnement (1, 2) en étant sous contrainte mécanique, un mors de serrage (23') du deuxième levier (2) pénétrant dans le canal en forme de U, et une limitation de déplacement, agissant entre les deuxième et troisième leviers, étant définie par une goupille d'arrêt (35) propre à être insérée séparément.

4. Instrument selon la revendication 3, **caractérisé en ce que** le ressort est remplacé par des éléments magnétiques (45, 46, 47), qui agissent les uns sur les autres par des forces d'attraction magnétique, au moins l'un de ces éléments magnétiques étant un aimant permanent (45) et l'autre ou les autres élément(s) magnétique(s) étant ferromagnétiques, l'aimant permanent ou l'un des aimants permanents étant disposé dans le premier levier d'actionnement (1) dans la zone située entre l'ouverture d'insertion (15) et l'articulation de levier (11), tandis que l'autre ou les autres éléments magnétiques est ou sont disposé(s) en des emplacements correspondants dans le deuxième levier d'actionnement (2) ou bien dans le troisième levier (3).

5. Instrument selon l'une des revendications 1 à 4, **caractérisé en ce que** la barre (20') présentant une élasticité à la flexion est disposée à côté d'une barre de référence (21), **en ce que** les deux barres s'étendent sensiblement parallèlement dans une direction et **en ce que**, transversalement à la direction des barres, des segments (20a, 21a) sont reliés aux barres et comportent des repères (209a, 209b, 211) pour la détermination de la déformation.

6. Instrument selon la revendication 5, **caractérisé en ce que** le segment (20a), situé sur la barre (20') présentant une élasticité à la flexion, est réalisé géométriquement de telle sorte que se produise une restriction de la flexion sous l'effet d'une coopération avec le segment (20b) situé sur la barre de référence (21).

7. Instrument selon l'une des revendications 1 à 7, **caractérisé en ce qu'**il est prévu, pour les moyens servant à mesurer la déformation de la barre, un capteur (8), notamment un capteur avec bandes de mesure d'allongement (8 ; 8a, 8b), et **en ce que** le capteur fait partie d'un appareil de mesure électrique et/ou électronique pour l'indication de la force de traction agissant dans l'élément de tension.

8. Instrument selon la revendication 7, **caractérisé en ce que** l'appareil de mesure est intégré au niveau du levier d'actionnement (2), sur lequel est disposé le capteur (8).

9. Instrument selon l'une des revendications 1 à 8, **caractérisé en ce que** l'ouverture d'insertion (15) du premier levier d'actionnement (1) est un oeil ou un interstice entre deux fourchons.

10. Instrument selon l'une des revendications 1 à 9, **caractérisé en ce que** les mors (23', 33') de la fente de serrage (32) présentent des nervures sur les surfaces de serrage (330), l'orientation des arêtes des nervures s'étendant de préférence transversalement à la direction de la force de traction.

11. Instrument selon l'une des revendications 1 à 7, **caractérisé en ce qu'**il est prévu, pour les moyens (20a, 21a ; 8) servant à mesurer la déformation de la barre, une indication du point zéro (209a), cette indication du point zéro étant réglable.
